# FASCICULE DE BREVET EUROPEEN

(11) **EP 0 714 627 B1**
(45) Date de publication et mention de la délivrance du brevet: **26.06.2002**
(21) Numéro de dépôt: 95402624.1
(22) Date de dépôt: 22.11.1995
(51) Int. Cl.: A61B 5/00, A61B 5/0428, A61B 5/04

(54) **Interface programmable pour appareil de type Holter pour l'enregistrement de signaux physiologiques, notamment d'activité cardiaque**
Programmierbares Interface für Holter-Instrument zur Aufzeichnung von physiologischen Signalen, insbesondere der Herztätigkeit
Programmable interface for Holter type apparatus for recording physiological signals, especially cardiac activity

(30) Priorité: 22.11.1994 FR 9413952
(43) Date de publication de la demande: 05.06.1996
(73) Titulaire: ELA MEDICAL (Société anonyme), F-92541 Montrouge (FR)
(72) Inventeur: Faisandier, Yves, F-75014 Paris (FR)
(74) Mandataire: Dupuis-Latour, Dominique

(56) Documents cités:
- EP-A- 0 581 073
- FR-A- 2 557 318

## Description

L'invention concerne, de façon générale, la surveillance à distance de paramètres physiologiques, notamment d'activité cardiaque, effectuée au moyen d'un enregistreur réalisant en continu et sur une longue période l'enregistrement de signaux physiologiques recueillis sur un patient.

Bien que l'invention soit décrite ici dans le cadre d'un enregistrement "Holter", c'est-à-dire de signaux cardiaques recueillis au moyen d'électrodes appliquées à un patient, cette application n'est pas limitative et l'invention peut être mise en oeuvre pour l'enregistrement d'autres types de signaux (tension artérielle, signaux électroencéphalographiques, etc.), et à des signaux recueillis par d'autres capteurs que des électrodes.

Dans de nombreux cas, il serait intéressant, avec ces appareils, d'étendre les mesures à d'autres capteurs (en complément ou en variante des capteurs pour lesquels est prévu l'appareil) ou, plus généralement, de pouvoir communiquer avec d'autres systèmes ambulatoires portés par le patient, ou implantés dans le patient.

On connaît divers procédés d'interfaçage tels que réseau, mini-réseau, protocole RS-232, etc., dont la diversité rend le choix difficile si l'on veut conserver une très grande variété des possibilités d'extension.

Par ailleurs, ces procédés d'interfaçage connus, qui reposent tous sur l'échange de données numériques, sont assez complexes à mettre en oeuvre lorsque l'on souhaite compléter le système par un ou plusieurs capteurs analogiques, avec en outre le risque d'une consommation élevée d'énergie, incompatible avec l'autonomie de la source d'énergie de l'appareil.

L'un des buts de la présente invention est de proposer une interface pour appareil de type Holter, destinée à recevoir des signaux provenant d'autres capteurs que les électrodes de recueil de l'activité cardiaque, ces signaux additionnels pouvant provenir de capteurs de nature (numérique ou analogique) très variée et en nombre quelconque (un ou plusieurs capteurs additionnels), mais au moyen d'une seule et même interface et sans qu'il soit nécessaire de modifier les circuits de l'appareil ou leur câblage interne.

L'invention vise également, outre le recueil de signaux par des capteurs supplémentaires, à procurer une possibilité de pilotage d'un système externe (appareil médical ambulatoire ou implanté, dispositif de communication, etc.) et ce avec la même interface que celle pouvant servir au recueil de signaux complémentaires.

À cet effet, essentiellement, l'invention propose de doter l'enregistreur Holter d'une interface mixte (analogique et numérique) rendue programmable par un circuit approprié permettant de la configurer en fonction des besoins particuliers simplement par des moyens logiques, sans modification de la configuration matérielle de l'appareil Holter.

Pour cela, l'appareil de l'invention qui est un enregistreur comportant des moyens processeurs d'un type connu, par exemple d'après le EP-A-0 578 530 (ELA Medical), comprend les éléments énoncés par la partie caractérisante de la revendication 1. Les sous-revendications visent des modes de mise en oeuvre avantageux particuliers.

D'autres caractéristiques et avantages de l'invention apparaîtront à la lecture de la description détaillée ci-dessous d'un exemple de réalisation, faite en référence aux dessins annexés.

La figure 1 est un schéma par blocs montrant la structure interne d'ensemble d'un enregistreur Holter incorporant les moyens de l'invention.

La figure 2 représente schématiquement un capteur analogique unique, relié à l'interface de l'invention.

La figure 3 représente schématiquement une pluralité de capteurs analogiques multiplexés, reliés à l'interface de l'invention.

La figure 4 représente schématiquement un capteur numérique relié à l'interface de l'invention.

La figure 5 représente schématiquement un système externe actif piloté par l'appareil Holter via l'interface de l'invention auquel il est relié.

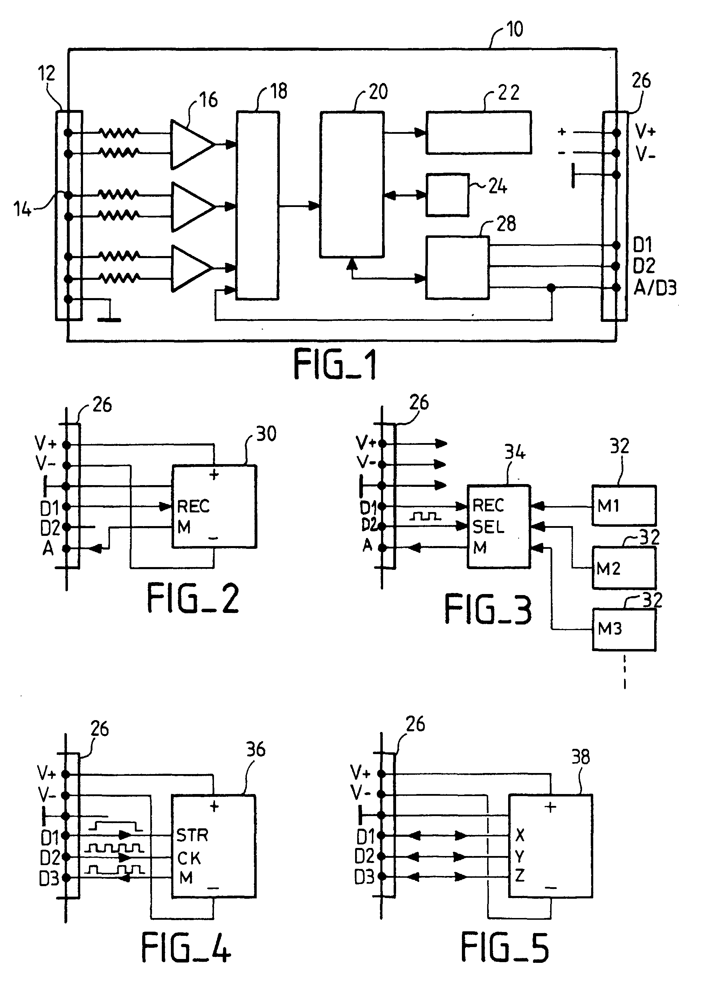

Sur la figure 1, la référence 10 désigne, de façon générale, un appareil de type enregistreur Holter, par exemple le modèle SYNESIS de la société ELA MÉDICAL. Cet appareil comprend un certain nombre de circuits, en eux-mêmes bien connus, permettant l'enregistrement de signaux physiologiques et la commande de l'appareil, et qui ne seront pas exposés en détail.

L'appareil 10 comporte un premier bornier ou prise 12 avec une pluralité de bornes 14, typiquement au nombre de sept (six voies de mesure et une masse). Chacune des bornes 14 est reliée, d'une part, à un câble de liaison à une électrode appliquée à un patient pour permettre le recueil de signaux physiologiques et, d'autre part, à une entrée d'un amplificateur 16 dont la sortie est appliquée à un convertisseur analogique/numérique 18 assurant la conversion, l'échantillonnage et le multiplexage des différents signaux issus des amplificateurs. Ces signaux sont ensuite traités par un circuit processeur 20, qui pilote un afficheur 22 et une mémoire de grande capacité 24.

Selon l'invention, l'appareil comporte un second bornier ou prise 26 pour une interface programmable vers des capteurs ou systèmes externes supplémentaires.

Dans le mode de réalisation illustré, la prise 26 comporte six bornes, à savoir :
- une borne de tension d'alimentation positive (V+), par exemple à + 2,5 volts,
- une borne d'alimentation négative (V-), par exemple à - 2,5 volts,
- une borne de masse,
- une première borne numérique (D1),
- une seconde borne numérique (D2), et
- une borne mixte analogique/numérique (A/D3).

Les trois bornes D1, D2 et A/D3 sont des bornes programmables reliées à un circuit logique 28 réalisant cette programmation, par exemple un circuit de type réseau logique programmable d'un type connu (par exemple un circuit XC3030 de la marque XLINX). Les portes contenues dans ce circuit sont programmées à la mise en route de l'appareil par transfert d'un ensemble de données depuis le circuit processeur 20 vers le réseau logique programmable 28.

Les signaux reçus sur les trois bornes programmables D1, D2, A/D3 sont appliqués par l'intermédiaire du circuit 28 au circuit processeur 20, où ils seront traités de manière appropriée ; inversement, les bornes D1, D2, D3 peuvent aussi faire fonction de bornes de sortie pour l'envoi de signaux de commande ou le pilotage d'un système externe ; elles reçoivent dans ce cas les signaux appropriés en provenance du circuit processeur 20 par l'intermédiaire du circuit 28.

La borne mixte A/D3, outre sa liaison au circuit 28 (fonction de borne numérique) est également reliée à une entrée analogique du convertisseur analogique/numérique 18, entrée à partir de laquelle le signal appliqué en entrée de la borne A/D3 pourra être traité de la. même manière que les signaux physiologiques délivrés par les amplificateurs 16. En ce qui concerne cette borne mixte A/D3, celle-ci sera désignée "A" dans la suite de la description lorsqu'elle fonctionnera en borne analogique, et "D3" lorsqu'elle fonctionnera en borne numérique ; dans ce dernier cas, on notera qu'elle peut être utilisée et programmée à volonté de la même manière que les deux autres bornes numériques D1 et D2 et ne présente alors aucun caractère spécifique par rapport à ces dernières.

Les figures 2 à 5 montrent diverses configurations de capteurs ou de systèmes susceptibles d'être connectés sur la prise 26 de l'appareil Holter, de manière à coopérer avec ce dernier grâce à l'interface de l'invention.

La figure 2 correspond au cas d'un capteur analogique unique 30 branché sur la prise 26.

Ce capteur 30 est alimenté par une tension (V+) ou deux tensions (V+ et V-), et la tension de sortie qu'il délivre sur la sortie de mesure M est envoyée directement sur la borne A, depuis laquelle cette tension de mesure sera appliquée en entrée du convertisseur analogique/numérique 18 de l'enregistreur Holter, qui va pouvoir traiter et éventuellement mémoriser le paramètre complémentaire délivré par le capteur additionnel 30.

Avantageusement, on utilise l'une des bornes numériques, par exemple la borne D1, comme borne de reconnaissance pour déterminer la présence ou non d'un capteur sur la prise 26.

A cet effet, l'appareil Holter, au moment de la mise en route et éventuellement à intervalles réguliers pour contrôle, envoie sur la borne D1, qui est normalement au niveau logique '0', un niveau '1' transmis à une entrée de reconnaissance REC du capteur ; ceci aura pour effet de forcer la sortie M du capteur à une valeur prédéterminée, par exemple une tension égale à la tension V+. Selon que cette tension apparaît ou non sur la borne A, l'appareil détermine alors si un capteur est ou non branché et adapte son fonctionnement en conséquence.

De façon plus générale, ce système de reconnaissance automatique peut être utilisé pour déterminer non seulement la présence ou l'absence d'un capteur ou d'un système sur la prise 26, mais également pour indiquer au système la configuration du capteur ou système branché : un simple '1' (tension V+) signifiera la présence d'un capteur analogique unique, tandis qu'un signal plus complexe tel qu'un mot de donnée numérique série signifiera la présence d'un système, (analogique ou numérique) plus complexe tel que ceux que l'on va maintenant exposer. L'appareil pourra ainsi automatiquement sélectionner la configuration appropriée du circuit programmable 28 en fonction du type de capteur ou système reconnu et initialiser dans le logiciel du circuit processeur 20 les différents paramètres correspondant à la configuration ainsi reconnue.

La figure 3 présente le cas d'une pluralité de capteurs analogiques 32, en nombre quelconque, délivrant des signaux analogiques sur leurs sorties respectives M1, M2, M3, ...

Un circuit intermédiaire 34 assure alors un rôle de multiplexage et de codage initial ; il comporte une entrée de reconnaissance REC, une entrée de sélection de voie SEL et une sortie multiplexée de mesure M, qui peut être une sortie analogique ou bien une sortie numérique, si le circuit intermédiaire 34 comprend lui-même un convertisseur analogique/numérique (de même nature que le convertisseur 18 décrit plus haut).

Les bornes V+ et éventuellement V- sont utilisées pour l'alimentation des capteurs 32 et du circuit 34.

L'application d'un niveau '1' sur la borne D1, transmis à la borne REC du circuit 34, permet la reconnaissance automatique, le circuit 34 délivrant sur sa sortie M un mot numérique série codé permettant de définir la nature du système analogique particulier raccordé à la prise 26. À partir de ces indications, l'appareil commande le multiplexage par envoi d'un mot numérique série codé sur la sortie D2, transmis à l'entrée de sélection SEL du circuit 34. La tension de sortie M*i* du capteur 32 sélectionné est transmise à la borne M du circuit 34 puis à l'entrée analogique A de l'appareil Holter et enfin au convertisseur analogique/numérique 18.

Cette configuration est par exemple intéressante lorsque l'on souhaite utiliser l'enregistreur Holter pour le recueil de signaux électroencéphalographiques (EEG), qui sont des signaux nécessitant une dizaine de voies de mesure, donc en trop grand nombre pour la prise conventionnelle 12, limitée à trois voies différentielles.

Sur la figure 4, on a illustré le cas d'un capteur numérique 36 branché sur la prise 26. Ce capteur comprend une entrée de commande d'échantillonnage STR, une sortie de mesure M et éventuellement une entrée de synchronisation CK. Ces trois bornes de capteur sont reliées respectivement aux bornes D1, D2 et D3 de la prise 26 de l'appareil Holter. Le capteur reçoit également les tensions d'alimentation V+ et V-.

Le capteur 36 délivre les données de mesure à l'entrée D3 sous forme d'un mot numérique série codé. Ces données sont transmises au circuit processeur 20 par l'intermédiaire du circuit 28.

Une extension à une pluralité de capteurs numériques est bien entendu possible, avec un schéma semblable avec celui du capteur unique. Les divers capteurs sont alors tous reliés en parallèle à la prise 26, par exemple par l'intermédiaire d'un bus de communication. Dans ce cas, le signal de demande d'échantillonnage STR est un code spécifique identifiant chacun des capteurs. Le capteur concerné, lorsqu'il reçoit le code d'identification qui lui est propre, transfère alors sa donnée sur la borne D3 via le bus, les sorties des autres capteurs restant à haute impédance en dehors des périodes d'émission (on utilise à cet effet une logique du type "trois-états"). L'appareil Holter doit bien entendu utiliser un processus d'identification capable d'éviter les collisions en gérant de manière appropriée les échanges de données entre appareil et capteurs. On peut en particulier utiliser à cet effet un standard de communication tel que le standard PHILIPS I²C.

Sur la figure 5, on a illustré le cas d'un système externe 38 actif piloté par l'appareil Holter via la prise 26. Les bornes X, Y et Z (entrées ou sorties) de ce système sont reliées aux bornes D1, D2 et D3, afin de gérer les échanges de données de la manière voulue et selon le type de système concerné. Ce système peut être, par exemple et bien entendu sans caractère limitatif, une pompe à drogue, une antenne de communication avec une prothèse implantable, un système de télétransmission des enregistrements Holter vers un site distant par un radio-modem, etc.

L'interface programmable de l'invention permet ainsi, par des moyens très simples, d'adjoindre à un appareil de type Holter classique une prise d'extension accroissant de façon très importante les domaines d'utilisation et les possibilités d'application d'un tel appareil.

## Revendications

1. Un appareil (10), notamment de type Holter, pour l'enregistrement de signaux physiologiques, notamment d'activité cardiaque, cet appareil comportant des moyens processeurs (20) et étant **caractérisé en ce qu'**il comporte, outre les bornes (14) de recueil des signaux physiologiques, une interface programmable comprenant :
- au moins une borne d'alimentation (V+, V-),
- au moins une borne de masse,
- au moins une borne numérique (D1, D2), coopérant avec lesdits moyens processeurs (20),
- au moins une borne mixte analogique/numérique (A/D3), d'une part reliée à une entrée de signal analogique de l'appareil, et d'autre part coopérant avec lesdits moyens processeurs, et
- un circuit logique (28) de programmation du mode de transfert des signaux par les bornes de l'interface.

2. L'appareil de la revendication 1, dans lequel le circuit logique est apte à programmer la borne mixte (A/D3) en une borne analogique de réception d'un signal analogique (M) de capteur (30, 32).

3. L'appareil de la revendication 1, dans lequel le circuit logique est apte à programmer la borne mixte (A/D3) en une borne numérique d'entrée de signal (M) de capteur (36) et/ou de sortie de signal (Z) de pilotage d'un circuit externe (38).

4. L'appareil de la revendication 1, dans lequel le circuit logique est apte à programmer au moins l'une desdites bornes numériques (D1, D2) en une borne d'entrée de signal de capteur et/ou de sortie de signal de pilotage d'un circuit externe (X, Y).

5. L'appareil de la revendication 1, dans lequel le circuit logique est apte à programmer au moins l'une desdites bornes numériques (D1) en une borne de reconnaissance de capteur émettant un signal d'interrogation (REC) vers ce capteur (30, 34) de manière que celui-ci, s'il est présent, force en réponse une autre borne de l'interface (A/D3) à une valeur prédéterminée.

6. L'appareil de la revendication 1, dans lequel le circuit logique est apte à programmer au moins l'une desdites bornes numériques (D1) en une borne émettant un code de sélection (SEL) vers un circuit (34) de multiplexage d'une pluralité de capteurs (32).

7. L'appareil de la revendication 1, dans lequel le circuit logique est apte à programmer au moins l'une desdites bornes numériques (D1) en une borne émettant un code de demande d'échantillonnage (STR) vers un capteur numérique (36).

8. L'appareil de la revendication 1, dans lequel le circuit logique est apte à programmer au moins l'une desdites bornes numériques (D2) en une borne émettant un signal d'horloge de synchronisation (CK) vers un capteur numérique (36).

9. L'appareil de la revendication 1, dans lequel le circuit logique de programmation (28) comprend un circuit de type réseau logique programmable.

## Patentansprüche

1. Ein Gerät (10), insbesondere vom Holter-Typ, zum Aufzeichnen physiologischer Signale, insbesondere von einer Herzaktivität, wobei dieses Gerät Prozessmittel (20) aufweist und **dadurch gekennzeichnet ist, dass** es außer den Anschlussklemmen (14) für ein Aufnehmen physiologischer Signale eine programmierbare Schnittstelle aufweist, welche aufweist:
- zumindest eine Versorgungsanschlussklemme (V+, V-),
- zumindest eine Erdungsanschlussklemme,
- zumindest eine numerische Anschlussklemme (D1, D2), welche mit den Prozessmitteln (20) zusammenwirkt,
- zumindest eine analog/numerisch gemischte Anschlussklemme (A/D3), die einerseits mit einem analogen Signaleingang des Geräts verbunden ist und andererseits mit den Prozessmitteln zusammenwirkt, und
- eine logische Schaltung (28) für eine Programmierung des Übertragungsmodus der Signale durch die Anschlussklemmen der Schnittstelle.

2. Gerät nach Anspruch 1, bei welchem die logische Schaltung dazu geeignet ist, die gemischte Anschlussklemme (A/D3) in eine analoge Anschlussklemme für einen Empfang eines Messfühlers (30, 32) zu programmieren.

3. Gerät nach Anspruch 1, bei welchem die logische Schaltung geeignet ist, um die gemischte Anschlussklemme (A/D3) in eine numerische Anschlussklemme eines Signaleingangs (M) eines Messfühlers (36) und/oder eines Signalsausgangs (Z) für eine Steuerung einer externen Schaltung (38) zu programmieren.

4. Gerät nach Anspruch 1, bei welchem die logische Schaltung geeignet ist, um zumindest eine der numerischen Anschlussklemmen (D1, D2) in eine Anschlussklemme eines Signaleingangs eines Messfühlers und/oder eines Signalausgangs für ein Steuern einer externen Schaltung (X, Y) zu programmieren.

5. Gerät nach Anspruch 1, bei welchem die logische Schaltung geeignet ist, um zumindest eine der numerischen Anschlussklemmen (D1) in eine Anschlussklemme für ein Erkennen eines Messfühlers zu programmieren, der ein Abfragesignal (REC) in Richtung dieses Messfühlers (30, 34) emittiert, auf eine Weise, dass dieser, wenn er vorhanden ist, in Antwort eine andere Anschlussklemme der Schnittstelle (A/D3) auf einen vorbestimmten Wert zwingt.

6. Gerät nach Anspruch 1, bei welchem die logische Schaltung geeignet ist, um zumindest eine der numerischen Anschlussklemmen (D1) in eine Anschlussklemme zu programmieren, die einen Auswahlcode (SEL) zu einer Schaltung (34) für ein Multiplexieren einer Mehrzahl von Messfühlern (32) aussendet.

7. Gerät nach Anspruch 1, bei welchem die logische Schaltung geeignet ist, um zumindest eine der numerischen Anschlussklemmen (D1) in eine Anschlussklemme zu programmieren, die einen Code für eine Anfrage einer Probennahme (STR) zu einem numerischen Messfühler (36) aussendet.

8. Gerät nach Anspruch 1, bei dem die logische Schaltung geeignet ist, um zumindest eine der numerischen Anschlussklemmen (D2) in eine Anschlussklemme zu programmieren, die ein Synchronisationstaktgebersignal (CK) zu einem numerischen Messfühler (36) aussendet.

9. Vorrichtung nach Anspruch 1, bei welcher die logische Schaltung für ein Programmieren (28) eine Schaltung vom Typ eines logisch programmierbaren Netzes aufweist.

## Claims

1. An appliance (10), in particular of the Holter type, for recording physiological signals, in particular of heart activity, said appliance including processor means (20) and being **characterized in that**, in addition to terminals (14) for picking up physiological signals, it further includes a programmable interface comprising:
· at least one power supply terminal (V+, V-);
· at least one ground terminal;
· at least one digital terminal (D1, D2) co-operating with said processor means (20);
· at least one combined analog and digital terminal (A/D3), connected firstly to an analog signal input of the appliance and secondly co-operating with said processor means; and
· a logic circuit (28) for programming the signal transfer mode via the terminals of the interface.

2. The appliance of claim 1, in which the logic circuit is suitable for programming the combined terminal (A/D3) to constitute an analog terminal for receiving an analog signal (M) from the sensor (30, 32).

3. The appliance of claim 1, in which the logic circuit is suitable for programming the combined terminal (A/D3) to constitute a digital input terminal for a signal (M) from a sensor (36) and/or an output terminal for a signal (Z) for controlling an external circuit (38).

4. The appliance of claim 1, in which the logic circuit is suitable for programming at least one of said digital terminals (D1, D2) to constitute an input terminal for a signal from a sensor and/or an output terminal for a signal for controlling an external circuit (X, Y).

5. The appliance of claim 1, in which the logic circuit is suitable for programming at least one of said digital terminals (D1) to constitute a terminal for recognizing a sensor sending an interrogation signal (REC) to the sensor (30, 34) in such a manner that if the sensor is present, it forces in response another terminal of the interface (A/D3) to take on a predetermined value.

6. The appliance of claim 1, in which the logic circuit is suitable for programming at least one of said digital terminals (D1) to constitute a terminal that sends a select code (SEL) to a circuit (34) for multiplexing a plurality of sensors (32).

7. The appliance of claim 1, in which said logic circuit is suitable for programming at least one of said digital terminals (D1) to constitute a terminal sending a sampling request code (STR) to a digital sensor (36).

8. The appliance of claim 1, in which the logic circuit is suitable for programming at least one of said digital terminals (D2) to constitute a terminal sending a synchronization clock signal (CK) to a digital sensor (36).

9. The appliance of claim 1, in which the programming logic circuit (28) comprises a circuit of the programmable logic array type.
